# EUROPEAN PATENT SPECIFICATION

(11) **EP 4 405 000 B1**
(45) Date of publication and mention of the grant of the patent: **01.10.2025**
(21) Application number: 22782727.6
(22) Date of filing: 22.09.2022
(51) Int. Cl.: A61M 5/178, G16H 20/17, A61M 5/315

(54) **OPERATING AN ELECTRONIC UNIT OF A DRUG DELIVERY DEVICE OR A DRUG DELIVERY ADD-ON DEVICE**
BETRIEB EINER ELEKTRONIKVORRICHTUNG EINER MEDIKAMENTENABGABEVORRICHTUNG ODER EINER WIRKSTOFFABGABEZUSATZVORRICHTUNG
FONCTIONNEMENT D'UN DISPOSITIF ÉLECTRONIQUE D'UN DISPOSITIF D'ADMINISTRATION DE MÉDICAMENTS OU UN DISPOSITIF COMPLÉMENTAIRE D'ADMINISTRATION DE MÉDICAMENTS

(30) Priority: 24.09.2021 EP 21315180
(43) Date of publication of application: 31.07.2024
(73) Proprietor: Sanofi, 75017 Paris (FR)
(72) Inventor: CORNE, Hester, Jane, Warwick, CV34 4AB (GB); SMITH, Ronald, Anthony, 8100-179 Loule (PT)
(74) Representative: McDougall, James
(86) International application number: PCT/EP2022/076294
(87) International publication number: WO 2023/046799

(56) References cited:
- US-A1- 2011 270 214
- US-A1- 2020 245 385
- US-B2- 9 931 037

## Description

### Field

The present disclosure relates to operating an electronic unit of a drug delivery device or a drug delivery add-on device.

### Background

WO2010/052275A2 describes an electronic drug delivery device, which optionally can be equipped with means for transferring data with an external device, where the drug delivery device incorporates a power-management method which is effective in minimizing power consumption for the incorporated electronic circuitry yet allows ease of use during operation of the device. In an exemplary embodiment the device has a low-power hibernating state in which two functions, e.g. detection and communication means, are in a low-power sleep state, a high-power state in which both of the functions, e.g. the detection and the communication means, are in an energized high-power state, and a medium-power state in which one function, e.g. the detection means, is in an energized high-power state and a second function, e.g. the communication means, are in a low-power sleep state.

WO2020/257137A1 describes adding communication functionality to a drug delivery device for purposes of transferring information to a user device (e.g., a mobile computing device such as a smartphone, a personal computer, a server, etc.) while maintaining power-efficient operation. In one embodiment, the drug delivery device comprises: a reservoir adapted to contain a drug, an injection mechanism coupled with the reservoir to deliver a drug from the reservoir, a power source, one or more sensors, a memory, a controller powered by the power source and having an active mode and a low-power mode. The controller is configured to, while operating in the active mode, use the one or more sensors to detect that the injection mechanism has performed an injection. The controller is also configured to generate in the memory a data entry indicative of the injection and/or a state of the drug delivery device, and switch into the low-power mode subsequent to or contemporaneous with detecting that the injection mechanism has performed the injection. The drug delivery device also comprises a wireless communication module powered by the power source and configured to establish a wireless connection with a user device while the controller is operating in the low-power mode, and transmit a message indicative of the injection and/or the state of the drug delivery device to the user device.

### Summary

This disclosure describes a method for operating an electronic unit of a drug delivery device or a drug delivery add-on device, an electronic unit of a drug delivery device or a drug delivery add-on device, and a drug delivery device or a drug delivery add-on device comprising such an electronic unit.

In one aspect the present disclosure provides a method for operating an electronic unit of a drug delivery device or a drug delivery add-on device, wherein the drug delivery device or the drug delivery add-on device comprise a dose measurement function and a data transmission function, and wherein the method comprises the following modes:
a shutdown mode, in which one or more components of the electronic unit are operated for minimum power consumption;
a dose capture and recording mode, in which one or more components of the electronic unit are operated to perform the dose measurement function;
a communication mode, in which one or more components of the electronic unit are operated to perform the data transmission function;
wherein the method operates the electronic unit as follows:
   in the shutdown mode, when the device is idle and/or after the dose capture and recording mode and/or after the communication mode;
   in the dose capture and recording mode upon receipt of a device reset or wake up signal;

in the communication mode upon initiation from the dose capture and recording mode. This method enables implementation in software allowing the electronic unit to be better adapted to the power management requirements and also enables implementation in the electronic unit, for example an implementation with several different controllers within the electronic unit each having different power requirements and being provided to implement different functionality. The shutdown mode may be the inactive state of the drug delivery device or drug delivery add-on device, in which nearly all components of the electronic unit, which are not required in the inactive state, may be powered down for minimum power consumption.

In embodiments, the method may further comprise a manufacturing mode, in which the electronic unit is operated for configuring and/or testing the device; and wherein the method operates the electronic unit as follows: in the manufacturing mode upon initiation from the dose capture and recording mode and receipt of a manufacturing mode request; in the shutdown mode, after the manufacturing mode. The manufacturing mode may enabled, for example, when the drug delivery device or add-on device comprising the electronic unit is manufactured. It also may enable diagnostics of a device, for example reading out settings and parameters.

In embodiments, the one or more components may comprise at least one processor, particularly a main controller and a sensor controller, and/or at least one communication interface component, particularly a Bluetooth^{®} communication module, and wherein the method may switch these components into different power modes having different power requirements depending on the operating mode of the electronic unit. The electronic unit may comprise a printed circuit board (PCB) with the components. Several components may be also implemented as a system on chip (SoC). A processor may be implemented by a microcontroller. Several processors may be implemented by a single microcontroller or SoC having multiple processor cores. The at least one communication interface component may implement different communication interfaces such as Bluetooth^{®}, ZigBee^{™}, Wi-Fi^{™} or NFC (Near Field Communication). The at least one communication interface component may be provided to implement connectivity for the drug delivery device or drug delivery add-on device, particularly with an external computing device such as a smartphone, a laptop, a tablet computer, a (cloud) server, a storage, a manufacturing test device or the like.

In embodiments, in the shutdown mode, the method may switch the at least one processor into a low power mode, in which its power requirement is minimal and it can be only woken by an external signal and contents stored in a volatile memory are lost, and the at least one communication interface component is powered off. The volatile memory may be for example an internal memory of the at least one processor and provided for temporarily storing parameters or data to be processed later so that its content may be lost when the shutdown mode is enabled. The low power mode can be a mode in which the at least one processor is in a kind of sleep state, in which most units of the processor are shut off to save energy, and only essential units are powered so that it can be woken by an external signal such as a hardware or software reset or dedicated wake up signal.

In embodiments, in the dose capture and recording mode, the method may switch the at least one processor, particularly the main controller, into a standby power mode, in which a captured dose stored in volatile memory is retained. In the standby power mode, the at least one processor may require more power than in the low power mode since a volatile memory remains powered. The volatile memory may be a processor internal memory, or a processor external memory supplied with signals from the processor.

In a further embodiment, the dose capture and recording mode may comprise time critical activities, which are performed before other activities and comprise a sequence of one or more of the following steps: initializing at least one processor, particularly the sensor controller and the main controller; checking for the receipt of the manufacturing mode request; dose capture with the at least one processor, particularly the sensor controller. By performing the time critical activities before other activities, the accuracy of does measurements may be increased since the method may more quickly perform dose measurement activities after leaving the shutdown mode.

In a yet further embodiment, the other activities may comprise a sequence of one or more of the following steps: reading a device battery voltage and a temperature; determining a device state; determining a reported dose; reading a real time counter value; determining a dose time; determining a flags value; generating at least one dose record; storing at least one dose record; arming a dose button of the device for a reset; determining a communication activity type; reading data from an external real time clock; generating an extended dose record; preparing shared date for transmission. These steps may particularly be performed for obtaining a complete dose record data set with, for example, an expelled dose, time, date, temperature, and for preparing a communication with an external computing device.

In embodiments, in the communication mode the method may switch the at least one processor, particularly the main controller, into a normal power mode, in which a captured dose stored in volatile memory can be stored in non-volatile memory, and the at least one communication interface component switched on in order to perform the data transmission function. This may prevent captured doses getting lost when the shutdown mode is entered and/or a data transmission of captured doses fails in that particular activation of the communication mode.

In embodiments, in the manufacturing mode, the method may switch the at least one communication interface component on in order to receive at least one message for configuring and/or testing the device. Thus, an automated test may be performed without requiring interaction since messages may be transmitted via the communication interface from, for example, a test station to a device under test in a production line. In embodiments, the at least one communication interface component may comprise a communication interface component dedicated for the manufacturing mode, for example a wired serial interface provided for the manufacturing mode, particularly for receiving and/or transmitting data such as messages related to the manufacturing mode. This communication interface component dedicated for the manufacturing mode may be implemented in an existing communication interface component, or provided in addition to a communication interface component such as a Bluetooth^{®} communication module.

In a further embodiment, the manufacturing mode may comprise a sequence of one or more of the following steps: initialising the manufacturing mode; waiting for a message; processing a received message; executing a command based on a processing of a received message; exiting the manufacturing mode after executing a command. For example, a message may comprise a test command for testing certain functions of the device under test and/or a command for setting parameters of the device under test.

In a further aspect the present disclosure provides a computer program executable by one or more processors and configuring the one or more processors to execute a method as disclosed herein. The computer program may be for example implemented as firmware stored in a non-volatile memory of the electronic unit, and may configure one or more processors of the electronic unit to implement the method as disclosed herein when executed by the one or more processors.

In a yet further aspect the present disclosure provides an electronic unit of a drug delivery device or a drug delivery add-on device, wherein the drug delivery device or the drug delivery add-on device comprises a dose measurement function and a data transmission function, and wherein the electronic unit is operable in different operating modes and comprises one or more components being capable of being be switched into different modes having different power requirements depending on the operating mode of the electronic unit, wherein the electronic unit comprises at least one processor and at least one non-volatile memory for storing a program configuring the at least one processor to execute a method as disclosed herein.

In embodiments, the one or more components may comprise at least one of the following: a sensor controller being provided for dose capture; a main controller being provided for dose recording and data transmission control; at least one communication interface component, particularly a Bluetooth^{®} communication module. The sensor controller may be, for example, a dedicated component provided for performing measurements when a dose is selected and expelled with the drug delivery device, and it may for example comprise only a memory for storing measurements values. The main controller may be, for example, a microcontroller with functionality for communicating with the sensor controller in order to control it and/or to obtain data from the sensor controller, such as measurements. The microcontroller may also comprise an internal volatile memory such as a random access memory (RAM) for temporarily storing data such as measurements received from the sensor controller and a non-volatile memory such as a read only memory (ROM) or flash memory for permanently storing data such as firmware and/or processed measurements or dose recordings.

In still further aspect the present disclosure provides a drug delivery device or a drug delivery add-on device comprising the electronic unit as disclosed herein. The drug delivery device may be for example an injection pen, the drug delivery add-on device for example a device being attachable to a drug delivery device such as to an injection pen.

### Brief Description of the Figures

Figure 1 shows an embodiment of a drug delivery device comprising integrated wireless data communication circuitry and external devices communicating with the drug delivery device;
Figure 2 shows an embodiment of a system comprising a drug delivery device, a wireless data communication accessory, and external devices communicating with the wireless data communication accessory;
Figure 3 shows a schematic block diagram of an embodiment of a device controller;
Figure 4 shows a block diagram of an embodiment of a software structure implementing a method for operating an electronic unit of a drug delivery device, for example, from Figure 1 or a drug delivery add-on device as shown in Figure 2;
Figure 5 shows a block diagram of an embodiment of operating software provided for a drug delivery device, for example, from Figure 1 or a drug delivery add-on device as shown in Figure 2;
Figures 6A and 6B show a flowchart of a dose capture and recording mode of an embodiment of the method for operating an electronic unit of a drug delivery device, for example, from Figure 1 or a drug delivery add-on device as shown in Figure 2;
Figure 7 shows a flowchart of a communication mode of an embodiment of the method for operating an electronic unit of a drug delivery device, for example, from Figure 1 or a drug delivery add-on device as shown in Figure 2;
Figure 8 shows a flowchart of a manufacturing mode of an embodiment of the method for operating an electronic unit of a drug delivery device, for example, from Figure 1 or a drug delivery add-on device as shown in Figure 2;
Figure 9 shows a sequence diagram of activities of a dose capture and recording mode of an embodiment of the method for operating an electronic unit of a drug delivery device; and
Figure 10 shows a sequence diagram of time critical activities of a dose capture and recording mode of an embodiment of the method for operating an electronic unit of a drug delivery device

### Detailed Description of Some Embodiments

In the following, embodiments of the present disclosure will be described with reference to injection devices, particularly an injection device in the form of a pen. The present disclosure is however not limited to such application and may equally well be deployed with other types of drug delivery devices, particularly with another shape than a pen. The concept underlying the embodiments of the present disclosure is generally applicable to any drug delivery device with a dual functionality requiring dose measurement by any means and resulting in transmission of this dose measurement by any means.

The functionality implemented using electronics and software in the below described injection devices with connectivity to external devices generally comprises the following aspects:
- dose capture and recording;
- processing of the measured dose information, particularly together with ancillary information such as battery voltage and any diagnostics;
- transmission of the dose information, particularly with other ancillary information, via a communication channel particularly established by a radio based communication connection.

The herein disclosed approaches for operating the electronic unit of a drug delivery device or drug delivery add-on device enable implemention of a software structure for an operating method of the device, and particularly enabling implemention of the above aspects.

Before embodiments of the herein disclosed approaches are described in detail, embodiments of drug delivery devices and drug delivery add-on devices with connectivity to external devices are described in detail. The electronic unit disclosed herein is particularly suited for integration into these devices since they usually use a one-time and often non-exchangeable battery, which should last the entire lifetime of the device or at least a predefined utilization time of 1 year or even longer. The batteries employed in these devices are usually button cells having a capacity measured in milliampere hours selected to supply an electric current sufficient to operate the electronic unit over the usual utilization time of the device, which may be the entire lifetime of the device, for example when the device is a disposable drug delivery device, or a predefined utilization time, for example when the device is a reusable drug delivery device.

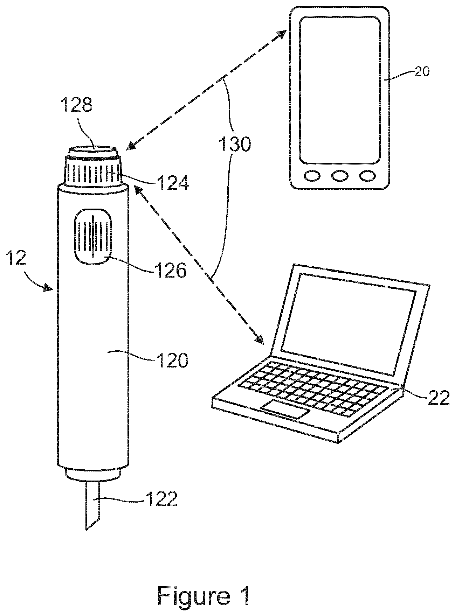

Figure 1 shows a drug delivery device 12 in the shape of an insulin injection pen, for example the reusable pen injector as described in WO2014033195 or the disposable pen injector as described in WO2004078239. The device 12 comprises an elongated body 120 having a pen-like shaped form for holding a drug cartridge and a dosage selection and delivery mechanism. At the lower end of the body 120, a syringe 122 is provided for expelling a drug dose and injecting this dose into a patient's body. The body 120 comprises at its other, upper, end a dial knob 124 for selecting a drug dose and an injection knob 128 for delivery of a selected dose. A user of the device 12 selects a dose by rotating the dial knob 124 around the longitudinal axis of the body 120. The selected dose is shown on a display 126 integrated in the body 120. After dose selection, the user may press the injection knob 128 in the direction of the longitudinal axis for expelling the selected dose via the syringe 122 into a patient's body. The dose selection and delivery mechanism contained in the body may comprise electronics (not visible in Figure 1) for detecting, storing and transmitting selected and delivered doses.

Wireless data communication circuitry is integrated in the device 12, which may be part of an electronics unit comprising wireless communication means for establishing a communication link 130 with an external device such as a smartphone 20 or a laptop computer 22, which may be paired with the wireless communication means. The term "paired" may mean that the wireless data communication circuitry and the external devices 20, 22 share some secret data such as cryptographic keys for establishing and/or securing data exchange.

The wireless data communication circuitry may be configured for establishing a short-range wireless communication link 130 via radio frequency communication such as a Bluetooth^{®} communication link and/or a Wi-Fi^{™} direct communication link based on the IEEE 802.11 standard (ISO/IEC 8802-11) with the external devices 20, 22 over a distance of at least several centimetres, particularly at least one meter, and more particularly several meters. The communication link 130 may be secured using data exchanged during the pairing process initially made for enabling the communication.

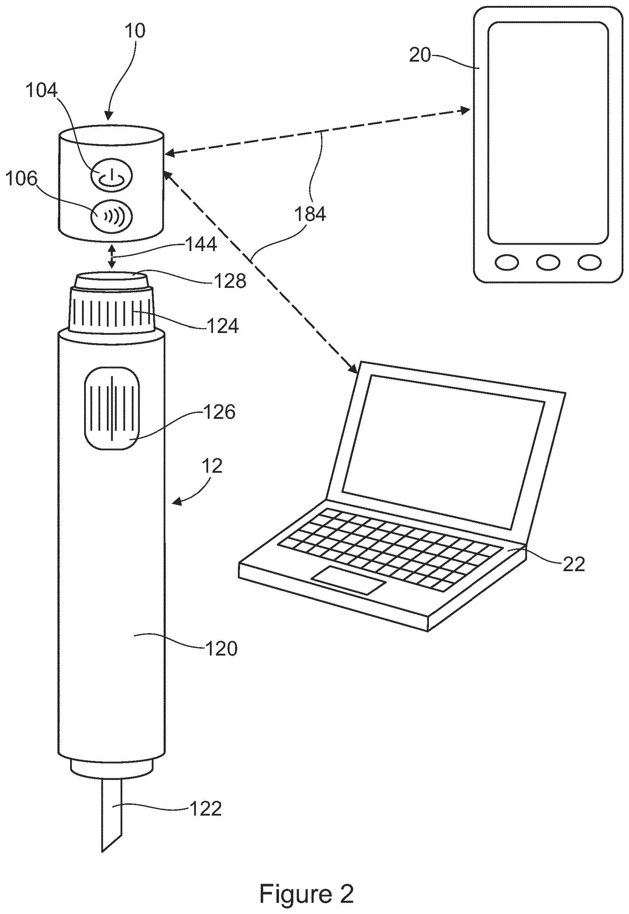

Figure 2 shows a system comprising a drug delivery device 12 in the shape of an insulin injection pen, for example the reusable pen injector as described in WO2014033195 or the disposable pen injector as described in WO2004078239, a drug delivery add-on device in the form of a wireless data communication accessory 10 attachable to the device 12, and an external device such as a smartphone 20 or a laptop computer 22.

The wireless data communication accessory 10 can be attached to the device 12 by clipping it onto the dial knob 124. The accessory 10 houses an electronic unit (not shown) comprising a first wireless communication means for establishing a first communication link 184 with an external device such as a smartphone 20 or a laptop computer 22, which may be paired with that wireless communication means, and comprising a second wireless communication means and/or wired communication means for establishing a second communication link 144 for exchanging data with a data exchange interface of the device 12.

The accessory 10 may comprise a power button 104 for activating and deactivating the power supply of the electronic unit manually. It may further comprise a wireless transmission button 106 provided for initiating a wireless transmission of data from the accessory to the external device 20 and/or 22. The button 106 may be also provided for initiating the pairing process of the accessory's 10 first wireless communication means with an external device 20, 22, for example by pressing the button 106 for a certain time period such as several seconds, thus switching the first wireless communication means into a pairing mode. The first wireless communication means can also be configured to establish automatically a communication link 184 with an already paired external device 20, 22 once the electronic unit of the accessory 10 is powered and the external device 20, 22 is within communication range of the first wireless communication means.

In embodiments, activating the power supply of the electronic unit of the accessory 10 may be performed automatically, i.e., without manually pressing the button 104, for example by means of an integrated switch of the accessory, which may be activated when the accessory is attached to the device 12 or when a dose is selected and/or delivered with the device 12. The integrated switch may be for example a mechanical switch or a magnetic switch, which may be activated when the accessory 10 is clipped on the dial knob 124 of the device 12 and/or when the dose selection and delivery mechanism is used by turning the dial knob 124 and/or pressing the injection knob 128.

The first communication means may be configured for establishing a short-range wireless communication such as Bluetooth^{®} 184 and/or a Wi-Fi^{™} communication link based on the IEEE 802.11 standard (ISO/IEC 8802-11) with the external devices 20, 22 over a distance of at least several centimetres, particularly at least one meter, and more particularly several meters. The maximum distance provided for communication may depend on the power supply available for the accessory 10. For example, when the accessory 10 is powered by a non-rechargeable battery, which should last several months, at least a year, or even longer, the maximum distance may be controlled by reducing the power requirements of the first communication means to meet the desired battery lifetime.

The second wireless communication means may be configured to establish a very-short-range wireless communication link employing inductive coupling for data transmission such as Near Field Communication (NFC) technology based on Radio-Frequency Identification (RFID) standards such as ISO/IEC 14443, FeliCa and/or ISO/IEC 18092.

For securing communication via the first wireless communication link 184, cryptographic information assigned to the drug delivery device 12 may be used. The cryptographic information may for example comprise some secret data such as one or more cryptographic keys, for example a symmetric encryption key or a public-private key pair for asymmetric encryption.

A pairing of the accessory 10 and drug delivery device 12 may be required for data exchange. The pairing may, for example, comprise receiving and storing some identification information from the drug delivery device 12 in a memory of the accessory 10, which, for example, may be used to tag data received from the drug delivery device 12 and/or to ensure that only data from paired devices 12 are read by the accessory.

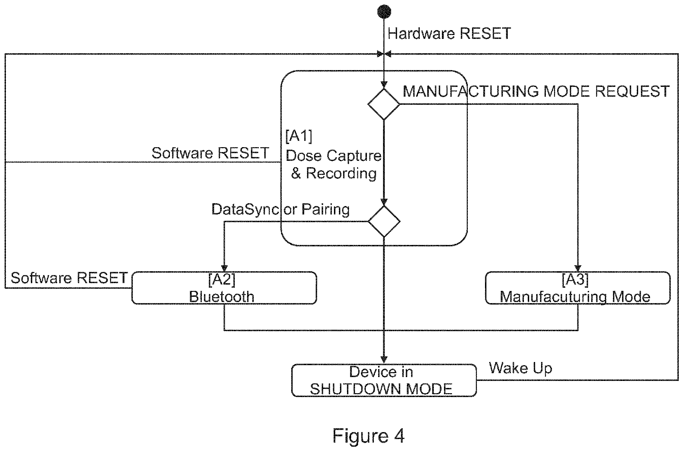

The device 12 or an add-on device 10 for attachment to the device 12 may also include a sensor unit 700, as shown schematically in Figure 4. The sensor unit 700 may comprise the sensor arrangement 215 including the two sensors 215a, 215b and an electronic unit for controlling the sensor arrangement 215 and performing other tasks such as communication with external devices, processing user inputs, outputting information for users etc. The sensor arrangement 215 may further comprise a sensor controller provided to control the operation of the sensors 215a, 215b. The electronic unit may comprise a processor arrangement 23 including one or more processors, such as a microprocessor, and/or a processor having multiple processing cores, particularly a main controller, a Digital Signal Processor (DSP), an Application Specific Integrated Circuit (ASIC), a Field Programmable Gate Array (FPGA), or the like, memory units 24, 25, including program memory 24 and main or working memory 25, which can store software and data for use by by the processor arrangement 23, a communication unit or output 27, which may be a wireless communications interface for communicating with another device via a wireless network such as Wi-Fi^{™} or Bluetooth^{®}, and/or an interface for a wired communications link, such as a socket for receiving a Universal Serial Bus (USB), mini-USB or micro-USB connector, a display unit 30, for example a Liquid Crystal Display (LCD), one or more Light Emitting Diodes (LEDs), and/or an electronic paper display, a user interface (UI) 31, for example one or more buttons and/or touch input devices, a power switch 28, and a battery 29. In embodiments, the program memory 24 and the main or working memory 25 can be also implemented by a single memory as in a von Neumann architecture, which contains both the software or program instructions to be executed by the processor arrangement 23 and the data, such as dose data. In embodiments, the memories 24 and/or 25 may be implemented as non-volatile memories, for example as Flash memory. In embodiments, one or both memories 24, 25 may be also implemented as volatile memory such as a RAM (Random Access Memory). In embodiments, a dedicated communication interface may be provided for a manufacturing mode (as described later), particularly for receiving and/or transmitting data related to the manufacturing mode. This dedicated interface may be for example implemented by the wireless and/or the wired interface, particularly as USB interface.

At least some of the components of the sensor unit 700 may exhibit or enable different power modes, particularly processors and/or controllers of the processor arrangement 23, the sensor arrangement 215, and the communication unit 27. The different power modes have different power requirements. An example of a power mode is a low power mode of a processor or controller in which the power requirement is minimal and the processor or controller can only be woken by an external signal and contents in a volatile memory such as a SRAM or DRAM are lost. Another example of a power mode is a standby power mode in which contents of a volatile memory are retained but most units of, for example, a processor or controller are switched off. In other low power modes a minimum functionality of the processor or controller may be maintained, for example, by reducing the clock frequency for data processing to reduce the power consumption. A further example of a power mode is the switching off a component such as the communication unit 27 when it is not used, for example when no connectivity is required. The control of the switching of components in different power modes may depend on the operating mode of the sensor unit 700 as will be described below in more detail.

The electronic unit components 23, 24, 25, 27, 28, 29, 30, 31 may be soldered onto a PCB containing the wiring between components. The sensor arrangement 215 may be also attached to the PCB or may be wired with the processor arrangement 23. The implementation of the sensor unit 700 depends on the drug delivery device or drug delivery add-on device, in which it should be integrated. For example, a PCB with the components 23, 24, 25, 27, 28, 29, 30, 31 may be integrated in the distal end of the injection device 12, and the sensors 215a, 215b may be arranged in the body 12 and connected to the PCB via wires. At least some of the components 23, 24, 25, 27 may be also comprised of a System on Chip (SoC) or microcontroller.

Firmware stored in the program memory 25 may configure the processor arrangement 23 to control the sensor arrangement 215 such that the expelling of a drug dose being delivered with the device 12 can be detected and the sensors 215a, 215b each output a sensor signal corresponding to the detected delivered drug dose. The processor arrangement 23 receives the sensor signal of each of the sensors 215a, 215b and takes readings of each sensor signal, which are processed to calculate the delivered dose. These tasks can be performed by the firmware configuring the sensor unit 700 in a dose capture and recording mode, which is described below in more detail. The firmware may further control communication via the communication unit 27 by operating the sensor unit 700 in a communication mode. In embodiments, the firmware can yet further operate the sensor unit 700 in a manufacturing mode, which may be provided for testing and/or configuration purposes of the device 12 or add-on device 10.

Figure 4 shows a block diagram of an embodiment of a software structure implementing a method for operating an electronic unit of a drug delivery device, for example, from Figure 1 or a drug delivery add-on device as shown in Figure 2. The software structure comprises three blocks for top level activities: A1 dose capture and recording mode, A2 Bluetooth^{®} communication mode, and A3 manufacturing mode. The relationship between these blocks and the transitions are as follows: the device's 10 or 12 inactive state is a SHUTDOWN MODE, in which nearly all components of the device's electronic unit, particularly the sensor unit 700, are powered off, or down, into a mode in which the power requirements are minimized. The SHUTDOWN MODE can be regarded as a low-level mode in contrast to the top level modes A1-A3. By utilising this low-level mode when no, or little, activity is required of the electronic unit the battery lifetime is maximized. When the device 10 or 12 is the SHUTDOWN MODE it may only be woken with a RESET signal, which may generated by, for example, pressing a DOSE BUTTON such as the injection knob 128 or by turning the dial knob 124 for selecting a drug dose, or by another source of RESET such as, for example, a hardware RESET or a software RESET from modes A1 or A2. When woken, the dose capture and recording mode A1 is first entered, and in this mode, it is first checked whether a manufacturing mode request was received to enter the manufacturing mode A3. If no manufacturing mode request was received, a dose may be captured particularly with the sensor arrangement 215 and recorded particularly in an internal memory of a processor or controller of the processor arrangement 23. After dose capture and recording a check may be made to determine if a data sync or pairing attempt should be initiated and if so the Bluetooth^{®} communication mode A2 may be entered.

Otherwise, the SHUTDOWN MODE is entered to switch the device 10 or 12 back into the low-level mode with a minimum power consumption. The SHUTDOWN MODE is also entered when the modes A2 and A3 are quit.

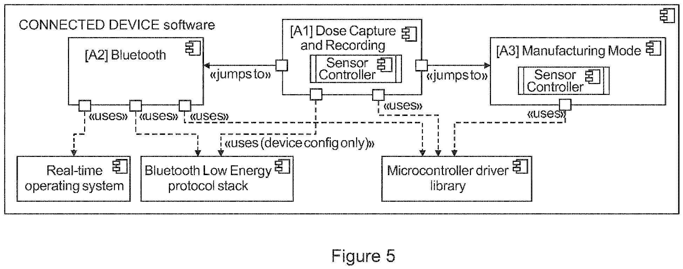

Figure 5 shows a block diagram of an embodiment of software provided for a drug delivery device, for example, from Figure 1 or a drug delivery add-on device as shown in Figure 2. The operating software is split into three top level software items implementing the different modes A1-A3 of the software structure shown in Figure 4. Each of the top-level software items uses at least one of the software items "Real time operating system" (RTOS), "Bluetooth^{®} Low Energy protocol stack", "Microcontroller driver library" as indicated by the arrows labelled with <<uses>>. The top-level items A1 and A3 use the sensor controller. The divergence from the item A1 to items A2 or A3 as shown in the block diagram from Figure 4 is indicated with the arrows labelled <<jumps to>>. This device (operating) software may be executed by one or more processors of the processor arrangement 23, particularly by a microcontroller of the processor arrangement 23.

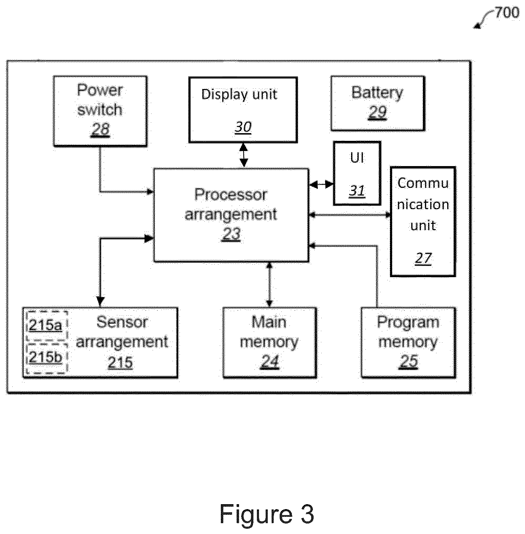

The software shown in Figures 4 and 5 may implement a power management strategy as described in the following: in order to maximise battery life the software remains in the SHUTDOWN MODE when the device 10 or 12 is idle. This is the lowest power mode supported by a processor or microcontroller of the electronic unit, particularly of the processor arrangement 23 of the sensor unit 700. Also, other components of the device's electronic unit, particularly the sensor unit 700, may be switched into their lowest power mode by the power management strategy of the software. From the lowest power mode, the processor or microcontroller can be woken with an external stimulus such as pressing a DOSE BUTTON, for example pressing the injection knob 128 or turning the dial knob 124 for selecting a drug dosage. In the SHUTDOWN MODE, only the contents of non-volatile memories such as ROM or Flash memories are retained, while the contents of volatile memories such as DRAM and SRAM are lost, and peripherals may be turned off, such as components 27, 30, 31 from the sensor unit 700 of Figure 3. When the dose capture and recording mode A1 is entered, i.e., the device 10 or 12 is woken up, the sensor controller may be activated, while the main core or processor or controller of the processor arrangement 23 may be placed into a standby mode. In the standby mode, contents of volatile memories are retained so that captured doses may be stored in a volatile memory. The power requirement in the standby mode is higher than in the lowest power mode, but lower than in the normal operating mode. When the sensor controller has completed its dose capture activities, it can wake up the main core or processor or controller of the processor arrangement so that it operates in the normal operating mode and can proceed with dose recording activities, which may comprise storing captured doses in a non-volatile memory together with additional data.

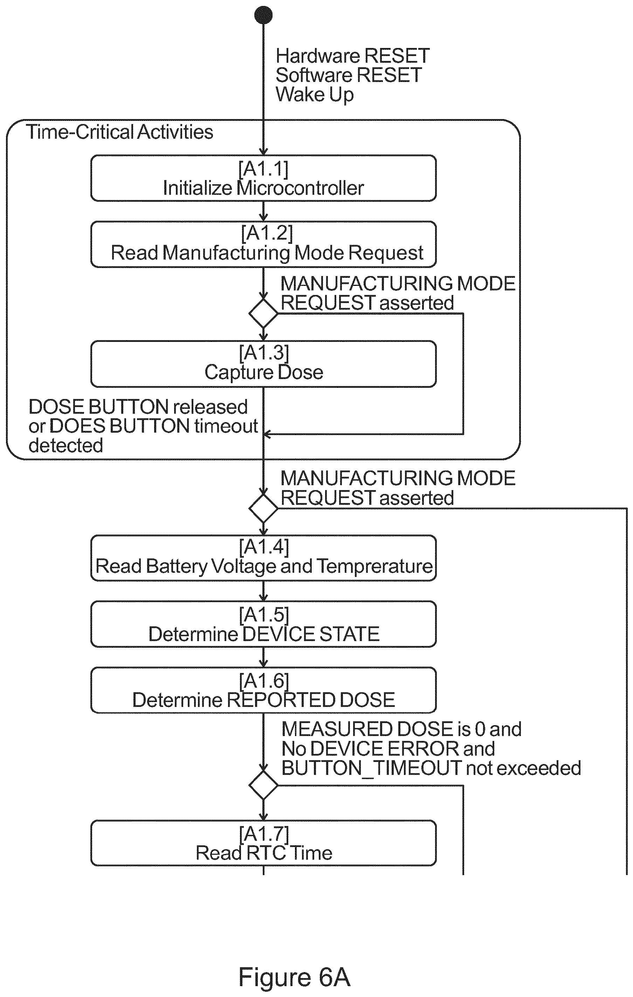

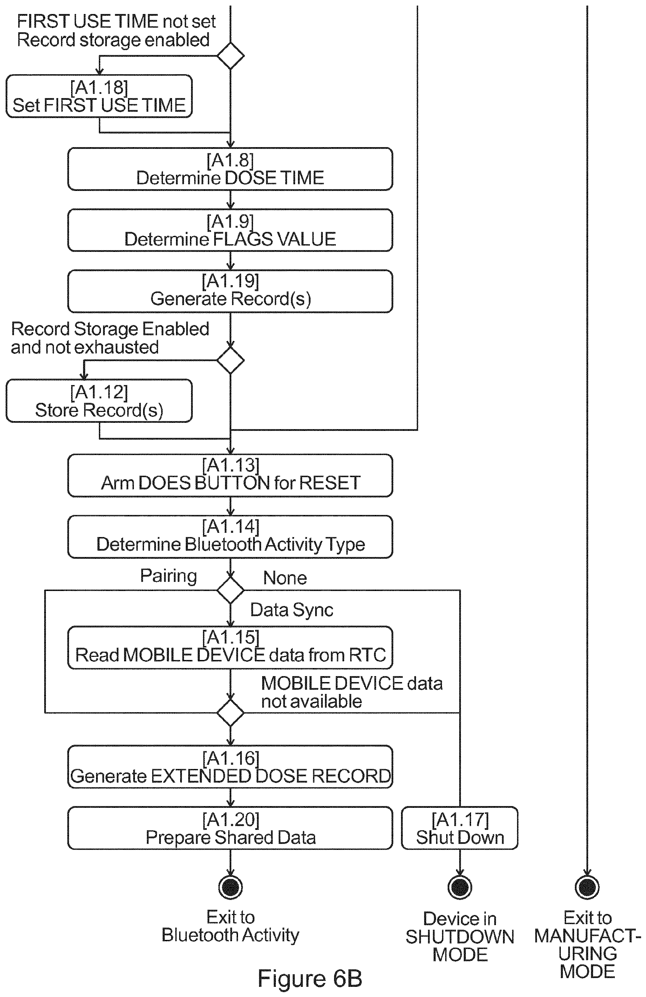

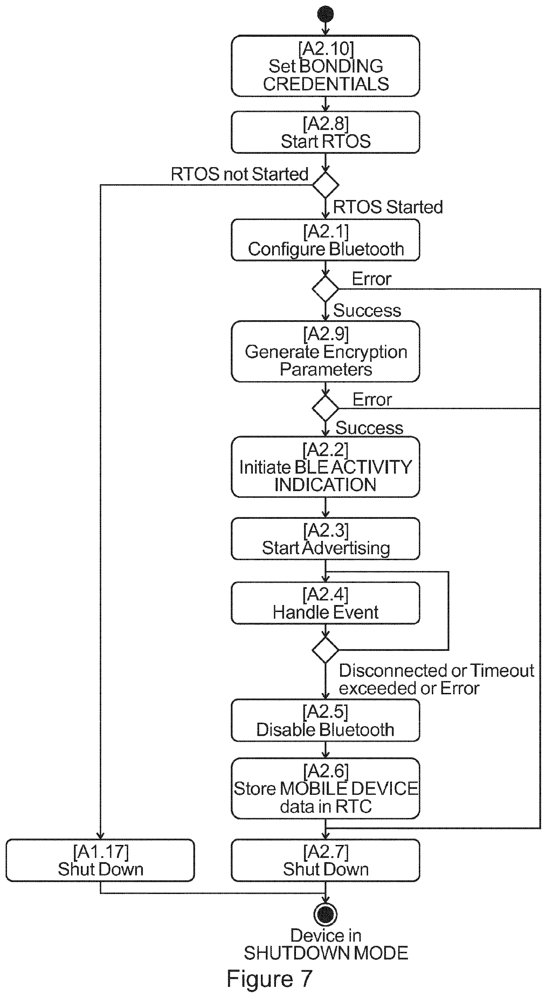

Figures 6A and 6B show a flowchart of a dose capture and recording mode of an embodiment of software implementing a method for operating an electronic unit such as the sensor unit 700 from Figure 3. The entry point of the mode is a Hardware RESET, a Software RESET or a Wake-up signal. The mode firstly carries out time critical activities in the following sequence: in step A1.1 a microcontroller or a processor is initialised; in a subsequent step A1.2 a manufacturing mode request is read, for example it is checked whether an input for a manufacturing mode request was received, for example a signal or flag is set; if no manufacturing mode request is asserted, the step A1.3 is executed to capture a dose, which may comprise powering on the sensor arrangement 215 and checking when a DOSE BUTTON is released or a DOSE BUTTON timeout is detected. Thus, step 1.3 may comprise all activities required for capturing the selecting and expelling of a dose including the control of the powering of the respective components required for dose capture such as the sensor arrangement 215 and receiving and storing dose measurement values from the sensor arrangement 215 in a volatile memory. After step A1.3, it is again checked whether a MANUFACTURING MODE REQUEST is asserted, and if not the procedure continues with step A1.4, in which the voltage of a battery of device 10 or 12 and/or a temperature value is/are read. In the subsequent step A1.5, the DEVICE STATE is determined, particularly if a device error is detected, and thereafter, the REPORTED DOSE is determined based on the dose measurement values in step A1.6. Then, it is checked whether a MEASURED DOSE is 0 and no DEVICE ERROR occurred, and a BUTTON TIMEOUT is not exceeded. If these conditions are not fulfilled, the procedure continues with step A1.7 for reading the time of a Real Time Clock (RTC). Thereafter, it is checked whether a flag FIRST USE TIME is not set and the record storage for dose records is enabled. If both conditions are met, the procedure continues with step A1.18 and sets the flag FIRST TIME USE before proceeding with step A1.8. Step A1.8 is carried out either directly after step A1.7 or after step A1.18 for determining the DOSE TIME based on the read RTC time. In the subsequent step A1.9, a FLAGS VALUE is determined, and thereafter a dose record with the determined DOSE TIME is generated in step A1.19. Thereafter, it is checked whether the record storage is enabled and not exhausted. If so, the generated dose record is stored in the record storage in step A1.12, otherwise the DOSE BUTTON is armed for RESET in step A1.13. In the subsequent step A1.14, the Bluetooth^{®} activity type is determined and thereafter checked if a pairing with an external device for establishing a Bluetooth^{®} communication connection is required, or a data sync should be carried out or nothing should be done. If nothing should be done, the procedure goes to step A1.17 to operate the device 10 or 12 in a shutdown mode. If a data sync should be carried out, the procedure continues with step A1.15, in which MOBILE DEVICE data are read from the RTC, and if no MOBILE DEVICE data are available, the procedure goes to step A1.17, and otherwise continues with step A1.16 for generating an EXTENDED DOSE RECORD followed by step A1.20 for preparing the data to be shared with an external device over a communication connection. After step A1.20, the procedure continues with operating the device 10 or 12 in the communication mode, which is shown in Figure 7 and described in the following.

Figure 7 shows a flowchart of a communication mode using Bluetooth^{®} as the communication means. First, BONDING CREDENTIALS are set in step A2.10. Thereafter, a Real Time Operating System (RTOS) is started to handle Bluetooth^{®} communication in step A2.8. If the RTOS is started, the procedure continues with step A2.1 and configures the Bluetooth^{®} communication. If the RTOS is not started, the procedure jumps to the step A1.17 to place the device 10 or 12 into shutdown mode. If step A2.1 ends without an error, the procedure continues with step A2.9 and generates encryption parameters. In case of an error after step A2.9, the procedure continues with step A2.7 to place the device 10 or 12 into shutdown mode. After step A2.9 without an error, step A2.2 is carried out to initiate a BLE ACTIVITY INDICATION, which is followed by step A2.3 to start advertising. In a subsequent step A2.4 event handling is carried out for communication. After step A2.4, it is checked whether the communication disconnected, timed out or an error occurred. If so, the procedure continues with step A2.5 and disables Bluetooth^{®}. Otherwise, the event handling in step A2.4 is repeated. After step A2.5, MOBILE DEVICE data are stored in the RTC in step A2.6, and thereafter the procedure continues with step A2.7.

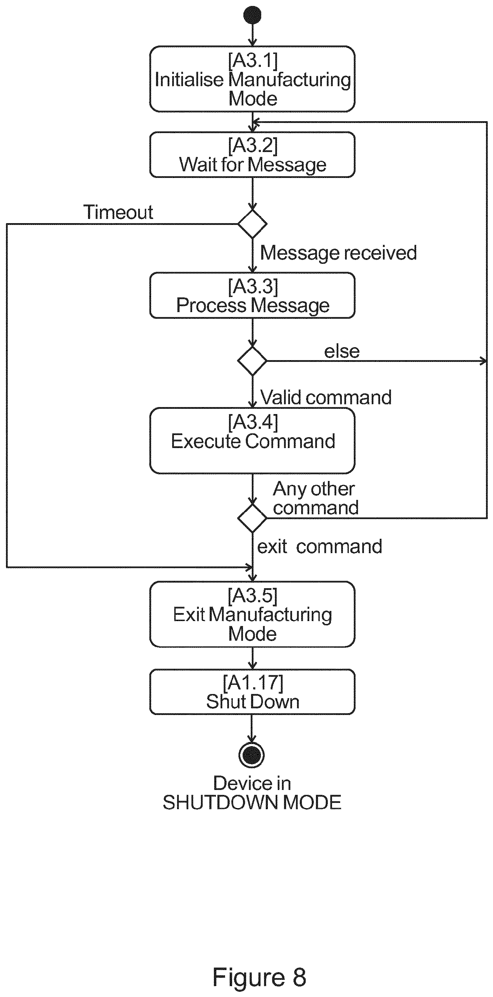

Figure 8 shows a flowchart of a manufacturing mode usable for configuring and testing the device 10 or 12 during manufacturing and for assessing the device 10 or 12 during a diagnostics investigation. The activities of the manufacturing mode as shown in the flowchart from Figure 8 are described in the following: in step A1.3, the manufacturing mode is initialised. Then, in step A3.2 the procedure waits for a message, for example a command from an external testing device to test the functionality of the device 10 or 12 under test. If no message is received within a predefined time span, a timeout occurs, and the procedure jumps to step A3.5 for exiting the manufacturing mode followed by step A1.17 to place the device 10 or 12 into shutdown mode. If a message is received within the predefined time span, the procedure continues with step A3.3 and processes the message, particularly one or more commands or one or more parameters contained in the message. If a contained command is valid, the procedure continues with step A3.4 and executes the command. Otherwise, the procedure returns to step A3.2. After step A3.4, the procedure checks whether another command is available and if so returns to step A3.2. If no other command is available, the procedure continues with step A3.5 for exiting the manufacturing mode followed by step A1.17.

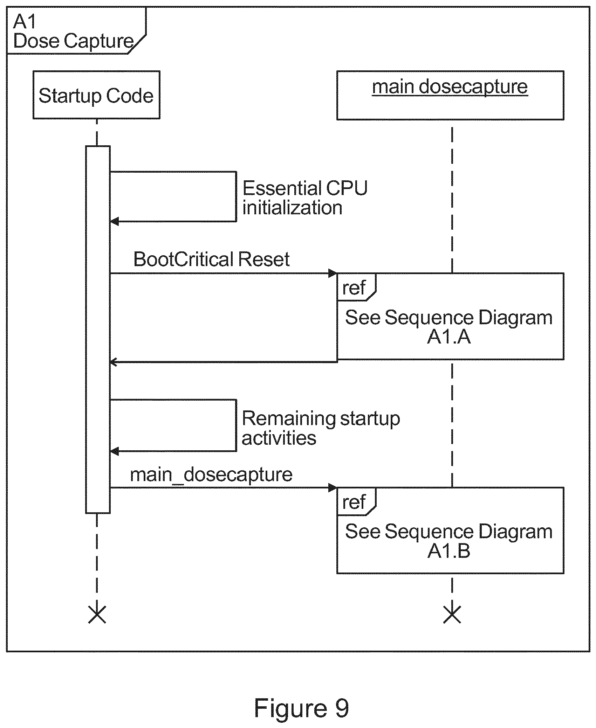

The time required for fully initialising the entire software system may be longer than the maximum allowable time between the DOSE BUTTON being pressed and a first Analog to Digital Conversion (ADC) reading or dose measurement value being taken as part of the dose capture activity. As a result, activities to support the fastest turnaround to dose measurement may be prioritized as time-critical activities within the dose capture and recording mode A1. This is shown in the sequence diagram of activities of an embodiment of the dose capture and recording mode A1 in Figure 9. The sequence diagram contains two sequences A1.A and A1.B.

Sequence A1.A relates to capturing the dose and is initiated after the essential processor or controller initialisation has taken place, but while there are some start up activities outstanding, including the initialisation of static data. Sequence A1.B relates to processing the captured dose and is initiated after the remaining start up activities have completed.

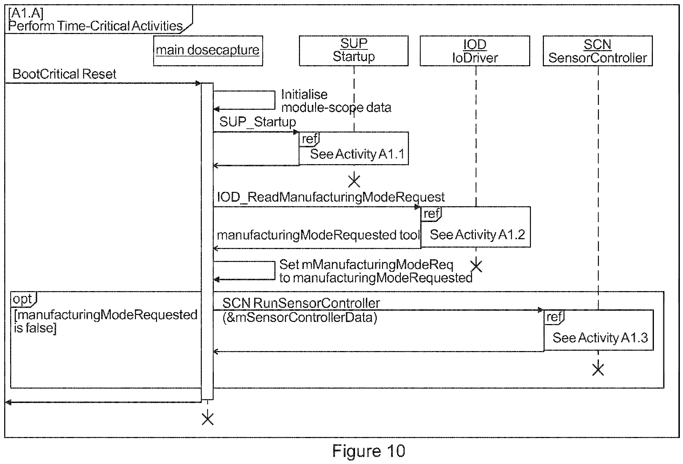

The sequence A1.A may comprise time-critical activities. The sequence diagram from Figure 10 shows how a BootCricitcalReset function, which may comprise time-critical activities, may be implemented. These time-critical activities can be performed by a sensor controller and a main controller being in a lower power state (since no processing power requiring functions may be required by these time-critical activities). So, the herein disclosed software structure allows optimisation of the power requirements by operating the device 10 or 12 in different modes, in which the components of the device's 10 or 12 electronic unit can be operated with different power requirements to fulfil required tasks. For example, as shown in Figures 9 and 10 by separating the time-critical activities from other activities of the dose capture and recording mode A1, it is not required to operate all components such as the sensor controller and the main controller in a normal power mode, but it may be sufficient to operate the sensor controller in normal power mode to perform the time-critical activities and the main controller in a lower power mode, particularly a standby power mode, in which only captured doses stored in a volatile memory for example of the main controller are retained, but other units of the main controller are powered down to save energy.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively, or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full-length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

## Claims

1. A method for operating an electronic unit (10) of a drug delivery device (12) or a drug delivery add-on device, wherein the drug delivery device or the drug delivery add-on device comprise a dose measurement function and a data transmission function, and wherein the method comprises the following modes:
- a shutdown mode, in which one or more components of the electronic unit are operated for minimum power consumption;
- a dose capture and recording mode (A1), in which one or more components of the electronic unit are operated to perform the dose measurement function;
- a communication mode (A2), in which one or more components of the electronic unit are operated to perform the data transmission function;
- wherein the method operates the electronic unit as follows:
∘ in the shutdown mode, when the device is idle and/or after the dose capture and recording mode (A1) and/or after the communication mode (A2);
∘ in the dose capture and recording mode (A1) upon receipt of a device reset or wake up signal;
∘ in the communication mode (A2) upon initiation from the dose capture and recording mode (A1).

2. The method of claim 1, further comprising a manufacturing mode (A3), in which the electronic unit is operated for configuring and/or testing the device; and wherein the method operates the electronic unit as follows:
- in the manufacturing mode (A3) upon initiation from the dose capture and recording mode (A1) and receipt of a manufacturing mode request;
- in the shutdown mode, after the manufacturing mode (A3).

3. The method of claim 1 or 2, wherein the one or more components comprise at least one processor (23), particularly a main controller and a sensor controller, and/or at least one communication interface component (27), particularly a Bluetooth^{®} communication module, and wherein the method switches these components into different power modes having different power requirements depending on the operating mode of the electronic unit.

4. The method of claim 3, wherein in the shutdown mode the method switches the at least one processor into a low power mode, in which its power requirement is minimal and it can be only woken by an external signal and contents stored in volatile memory (24, 25) are lost, and the at least one communication interface component is powered off.

5. The method of claim 3 or 4, wherein in the dose capture and recording mode (A1) the method switches the at least one processor, particularly the main controller, into a standby power mode, in which a captured dose stored in volatile memory is retained.

6. The method of claim 5, wherein the dose capture and recording mode (A1) comprises time critical activities (A1.1-A1.3), which are performed before other activities and comprise a sequence of one or more of the following steps: initializing at least one processor (A1.1), particularly the sensor controller and the main controller; checking for the receipt of the manufacturing mode request (A1.2); dose capture (A1.3) with the at least one processor, particularly the sensor controller.

7. The method of claim 6, wherein the other activities comprise a sequence of one or more of the following steps: reading a device battery voltage and a temperature (A1.4); determining a device state (A1.5); determining a reported dose (A1.6); reading a real time counter value (A1.7); determining a dose time (A1.8); determining a flags value (A1.9); generating at least one dose record (A1.19); storing at least one dose record (A1.12); arming a dose button of the device for a reset (A1.13); determining a communication activity type (A1.14); reading data from an external real time clock (A1.15); generating an extended dose record (A1.16); preparing shared date for transmission (A1.20).

8. The method of claim 3 to 7, wherein in the communication mode (A2) the method switches the at least one processor, particularly the main controller, into a normal power mode, in which a captured dose stored in volatile memory can be stored in non-volatile memory, and the at least one communication interface component is switched on in order to perform the data transmission function.

9. The method of any of claims 3 to 8, wherein in the manufacturing mode (A3) the method switches the at least one communication interface component on in order to receive at least one message for configuring and/or testing the device.

10. The method of claim 9, wherein the manufacturing mode (A3) comprises a sequence of one or more of the following steps: initialising the manufacturing mode (A3.1); waiting for a message (A3.2); processing a received message (A3.3); executing a command based on a processing of a received message (A3.4); exiting the manufacturing mode after executing a command (A3.5).

11. A computer program executable by one or more processors (23) and configuring the one or more processors to execute a method of any preceding claim.

12. An electronic unit (20) of a drug delivery device (12) or a drug delivery add-on device, wherein the drug delivery device or the drug delivery add-on device comprises a dose measurement function and a data transmission function, and wherein the electronic unit is operable in different operating modes and comprises one or more components being capable of being switched into different modes having different power requirements depending on the operating mode of the electronic unit, wherein the electronic unit comprises at least one processor (23) and at least one non-volatile memory (24, 25) for storing a program configuring the at least one processor to execute a method of any of claims 1 to 10.

13. The electronic unit of claim 12, wherein the one or more components comprise at least one of the following:
- a sensor controller being provided for dose capture;
- a main controller being provided for dose recording and data transmission control;
- at least one communication interface component (27), particularly a Bluetooth^{®} communication module.

14. A drug delivery device (12) or a drug delivery add-on device comprising the electronic unit (10) of any of claims 12 or 13.

## Patentansprüche

1. Verfahren zum Betreiben einer elektronischen Einheit (10) einer Arzneimittelabgabevorrichtung (12) oder einer Arzneimittelabgabezusatzvorrichtung, wobei die Arzneimittelabgabevorrichtung oder die Arzneimittelabgabezusatzvorrichtung eine Dosismessfunktion und eine Datenübertragungsfunktion umfasst und wobei das Verfahren die folgenden Modi umfasst:
- einen Abschaltmodus, in dem eine oder mehrere Komponente (n) der elektronischen Einheit für minimalen Stromverbrauch betrieben werden;
- einen Dosiserfassungs- und -aufzeichnungsmodus (A1), in dem eine oder mehrere Komponente (n) der elektronischen Einheit betrieben werden, um die Dosismessfunktion durchzuführen;
- einen Kommunikationsmodus (A2), in dem eine oder mehrere Komponente(n) der elektronischen Einheit betrieben werden, um die Datenübertragungsfunktion durchzuführen;
- wobei das Verfahren die elektronische Einheit wie folgt betreibt:
∘ in dem Abschaltmodus, wenn die Vorrichtung inaktiv ist, und/oder nach dem Dosiserfassungs- und - aufzeichnungsmodus (A1) und/oder nach dem Kommunikationsmodus (A2);
∘ in dem Dosiserfassungs- und -aufzeichnungsmodus (A1) nach einem Empfang eines Vorrichtungsrücksetz- oder -wecksignals;
∘ in dem Kommunikationsmodus (A2) nach einer Initiierung aus dem Dosiserfassungs- und - aufzeichnungsmodus (A1).

2. Verfahren nach Anspruch 1, ferner umfassend einen Fertigungsmodus (A3), in dem die elektronische Einheit zum Konfigurieren und/oder Testen der Vorrichtung betrieben wird; und wobei das Verfahren die elektronische Einheit wie folgt betreibt:
- in dem Fertigungsmodus (A3) nach einer Initiierung aus dem Dosiserfassungs- und -aufzeichnungsmodus (A1) und einem Empfang einer Fertigungsmodusanforderung;
- in dem Abschaltmodus nach dem Fertigungsmodus (A3).

3. Verfahren nach Anspruch 1 oder 2, wobei die eine oder die mehreren Komponente(n) mindestens einen Prozessor (23), insbesondere eine Hauptsteuerung und eine Sensorsteuerung, und/oder mindestens eine Kommunikationsschnittstellenkomponente (27), insbesondere ein Bluetooth^{®}-Kommunikationsmodul, umfasst/umfassen und wobei das Verfahren diese Komponenten abhängig vom Betriebsmodus der elektronischen Einheit in verschiedene Leistungsmodi mit verschiedenen Leistungsanforderungen schaltet.

4. Verfahren nach Anspruch 3, wobei das Verfahren im Abschaltmodus den mindestens einen Prozessor in einen Niedrigleistungsmodus schaltet, in dem seine Leistungsanforderung minimal ist und er nur durch ein externes Signal geweckt werden kann und Inhalte, die in einem flüchtigen Speicher (24, 25) gespeichert sind, verloren gehen, und die mindestens eine Kommunikationsschnittstellenkomponente ausgeschaltet wird.

5. Verfahren nach Anspruch 3 oder 4, wobei das Verfahren in dem Dosiserfassungs- und - aufzeichnungsmodus (A1) den mindestens einen Prozessor, insbesondere die Hauptsteuerung, in einen Standby-Leistungsmodus schaltet, in dem eine erfasste Dosis, die in einem flüchtigen Speicher gespeichert ist, erhalten bleibt.

6. Verfahren nach Anspruch 5, wobei der Dosiserfassungs- und -aufzeichnungsmodus (A1) zeitkritische Aktivitäten (A1.1 - A1.3) umfasst, die vor anderen Aktivitäten durchgeführt werden und eine Sequenz eines oder mehrerer der folgenden Schritte umfassen: Initialisieren mindestens eines Prozessors (A1.1), insbesondere der Sensorsteuerung und der Hauptsteuerung; Prüfen auf den Empfang der Fertigungsmodusanforderung (A1.2); Dosiserfassung (A1.3) mit dem mindestens einen Prozessor, insbesondere der Sensorsteuerung.

7. Verfahren nach Anspruch 6, wobei die anderen Aktivitäten eine Sequenz eines oder mehrerer der folgenden Schritte umfassen: Lesen einer Vorrichtungsbatteriespannung und einer Temperatur (A1.4); Bestimmen eines Vorrichtungszustands (A1.5); Bestimmen einer berichteten Dosis (A1.6); Lesen eines Echtzeitzählerwerts (A1.7); Bestimmen einer Dosiszeit (A1.8); Bestimmen eines Flag-Werts (A1.9); Erzeugen mindestens einer Dosisaufzeichnung (A1.19); Speichern mindestens einer Dosisaufzeichnung (A1.12); Freigeben einer Dosistaste der Vorrichtung für einen Rücksetzen (A1.13); Bestimmen eines Kommunikationsaktivitätstyps (A1.14); Lesen von Daten von einer externen Echtzeituhr (A1.15); Erzeugen einer erweiterten Dosisaufzeichnung (A1.16); Erstellen eines gemeinsamen Datums zur Übertragung (A1.20).

8. Verfahren nach Anspruch 3 bis 7, wobei das Verfahren in dem Kommunikationsmodus (A2) den mindestens einen Prozessor, insbesondere die Hauptsteuerung, in einen normalen Leistungsmodus schaltet, in dem eine in einem flüchtigen Speicher gespeicherte erfasste Dosis in einem nichtflüchtigen Speicher gespeichert werden kann, und die mindestens eine Kommunikationsschnittstellenkomponente eingeschaltet wird, um die Datenübertragungsfunktion durchzuführen.

9. Verfahren nach einem der Ansprüche 3 bis 8, wobei das Verfahren in dem Fertigungsmodus (A3) die mindestens eine Kommunikationsschnittstellenkomponente einschaltet, um mindestens eine Nachricht zum Konfigurieren und/oder Testen der Vorrichtung zu empfangen.

10. Verfahren nach Anspruch 9, wobei der Fertigungsmodus (A3) eine Sequenz eines oder mehrerer der folgenden Schritte umfasst: Initialisieren des Fertigungsmodus (A3.1); Warten auf eine Nachricht (A3.2); Verarbeiten einer empfangenen Nachricht (A3.3); Ausführen eines Befehls basierend auf einer Verarbeitung einer empfangenen Nachricht (A3.4); Verlassen des Fertigungsmodus nach einem Ausführen eines Befehls (A3.5).

11. Computerprogramm, das durch den einen oder die mehreren Prozessor(en) (23) ausführbar ist und den einen oder die mehreren Prozessor(en) dazu konfiguriert, ein Verfahren nach einem der vorstehenden Ansprüche auszuführen.

12. Elektronische Einheit (20) einer Arzneimittelabgabevorrichtung (12) oder einer Arzneimittelabgabezusatzvorrichtung, wobei die Arzneimittelabgabevorrichtung oder die Arzneimittelabgabezusatzvorrichtung eine Dosismessfunktion und eine Datenübertragungsfunktion umfasst und wobei die elektronische Einheit in verschiedenen Betriebsmodi betreibbar ist und eine oder mehrere Komponente(n) umfasst, die fähig sind, in verschiedene Modi mit verschiedenen Leistungsanforderungen abhängig vom Betriebsmodus der elektronischen Einheit geschaltet zu werden, wobei die elektronische Einheit mindestens einen Prozessor (23) und mindestens einen nichtflüchtigen Speicher (24, 25) zum Speichern eines Programms, das den mindestens einen Prozessor dazu konfiguriert, ein Verfahren nach einem der Ansprüche 1 bis 10 auszuführen, umfasst.

13. Elektronische Einheit nach Anspruch 12, wobei die eine oder die mehreren Komponente(n) mindestens eines der Folgenden umfasst/umfassen:
- eine Sensorsteuerung, die zur Dosiserfassung bereitgestellt ist;
- eine Hauptsteuerung, die zur Steuerung der Dosisaufzeichnung und der Datenübertragung bereitgestellt ist;
- mindestens eine Kommunikationsschnittstellenkomponente (27), insbesondere ein Bluetooth^{®}-Kommunikationsmodul.

14. Arzneimittelabgabevorrichtung (12) oder Arzneimittelabgabezusatzvorrichtung, umfassend die elektronische Einheit (10) nach einem der Ansprüche 12 oder 13.

## Revendications

1. Procédé destiné à faire fonctionner une unité électronique (10) d'un dispositif d'administration de médicament (12) ou d'un dispositif complémentaire d'administration de médicament, le dispositif d'administration de médicament ou le dispositif complémentaire d'administration de médicament comprenant une fonction de mesure de dose et une fonction de transmission de données, et le procédé comprenant les modes suivants :
- un mode arrêt, dans lequel un ou plusieurs composants de l'unité électronique fonctionnent pour une consommation d'énergie minimale ;
- un mode capture et enregistrement de dose (A1), dans lequel un ou plusieurs composants de l'unité électronique fonctionnent pour réaliser la fonction de mesure de dose ;
- un mode communication (A2), dans lequel un ou plusieurs composants de l'unité électronique fonctionnent pour réaliser la fonction de transmission de données ;
- le procédé faisant fonctionner l'unité électronique comme suit :
∘ dans le mode arrêt, lorsque le dispositif est inactif et/ou après le mode capture et enregistrement de dose (A1) et/ou après le mode communication (A2) ;
∘ dans le mode capture et enregistrement de dose (A1) à la réception d'un signal de réinitialisation ou de réveil de dispositif ;
∘ dans le mode communication (A2) lors d'un lancement à partir du mode capture et enregistrement de dose (A1).

2. Procédé de la revendication 1, comprenant en outre un mode fabrication (A3), dans lequel l'unité électronique fonctionne pour configurer et/ou tester le dispositif ; et le procédé faisant fonctionner l'unité électronique comme suit :
- dans le mode fabrication (A3) lors d'un lancement à partir du mode capture et enregistrement de dose (A1) et à la réception d'une demande de mode fabrication ;
- dans le mode arrêt, après le mode fabrication (A3).

3. Procédé de la revendication 1 ou 2, le ou les composants comprenant au moins un processeur (23), en particulier un dispositif de commande principal et un dispositif de commande de capteur, et/ou au moins un composant d'interface de communication (27), en particulier un module de communication Bluetooth^{®}, et le procédé faisant basculer ces composants dans différents modes d'alimentation ayant des besoins en énergie différents en fonction du mode de fonctionnement de l'unité électronique.

4. Procédé de la revendication 3, le procédé, dans le mode arrêt, faisant basculer l'au moins un processeur dans un mode économie d'énergie, dans lequel son besoin en énergie est minimal et il peut être réveillé uniquement par un signal externe et le contenu stocké dans une mémoire volatile (24, 25) est perdu, et l'au moins un composant d'interface de communication est mis hors tension.

5. Procédé de la revendication 3 ou 4, le procédé, dans le mode capture et enregistrement de dose (A1), faisant basculer l'au moins un processeur, en particulier le dispositif de commande principal, dans un mode d'alimentation en veille, dans lequel une dose capturée stockée dans une mémoire volatile est conservée.

6. Procédé de la revendication 5, dans lequel le mode capture et enregistrement de dose (A1) comprend des activités prioritaires (A1.1 à A1.3), qui sont effectuées avant d'autres activités et comprennent une séquence d'une ou plusieurs des étapes suivantes : initialisation d'au moins un processeur (A1.1), en particulier du dispositif de commande de capteur et du dispositif de commande principal ; vérification de la réception de la demande de mode fabrication (A1.2) ; capture de dose (A1.3) avec l'au moins un processeur, en particulier le dispositif de commande de capteur.

7. Procédé de la revendication 6, dans lequel les autres activités comprennent une séquence d'une ou plusieurs des étapes suivantes : lecture d'une tension de batterie de dispositif et d'une température (A1.4) ; détermination d'un état de dispositif (A1.5) ; détermination d'une dose rapportée (A1.6) ; lecture d'une valeur de compteur en temps réel (A1.7) ; détermination d'une heure de dose (A1.8) ; détermination d'une valeur d'indicateurs (A1.9) ; génération d'au moins un enregistrement de dose (A1.19) ; stockage d'au moins un enregistrement de dose (A1.12) ; armement d'un bouton de dose du dispositif pour une réinitialisation (A1.13) ; détermination d'un type d'activité de communication (A1.14) ; lecture de données à partir d'une horloge en temps réel externe (A1.15) ; génération d'un enregistrement de dose prolongée (A1.16) ; préparation de données partagées pour une transmission (A1.20).

8. Procédé des revendications 3 à 7, le procédé, dans le mode communication (A2), faisant basculer l'au moins un processeur, en particulier le dispositif de commande principal, dans un mode d'alimentation normale, dans lequel une dose capturée stockée dans une mémoire volatile peut être stockée dans une mémoire non volatile, et l'au moins un composant d'interface de communication est mis sous tension afin de réaliser la fonction de transmission de données.

9. Procédé de l'une quelconque des revendications 3 à 8, le procédé, dans le mode fabrication (A3), mettant sous tension l'au moins un composant d'interface de communication afin de recevoir au moins un message destiné à configurer et/ou tester le dispositif.

10. Procédé de la revendication 9, dans lequel le mode fabrication (A3) comprend une séquence d'une ou plusieurs des étapes suivantes : initialisation du mode fabrication (A3.1) ; attente d'un message (A3.2) ; traitement d'un message reçu (A3.3) ; exécution d'une commande basée sur un traitement d'un message reçu (A3.4) ; sortie du mode fabrication après exécution d'une commande (A3.5).

11. Programme informatique exécutable par un ou plusieurs processeurs (23) et configurant le ou les processeurs pour exécuter un procédé d'une quelconque revendication précédente.

12. Unité électronique (20) d'un dispositif d'administration de médicament (12) ou d'un dispositif complémentaire d'administration de médicament, le dispositif d'administration de médicament ou le dispositif complémentaire d'administration de médicament comprenant une fonction de mesure de dose et une fonction de transmission de données, et l'unité électronique pouvant fonctionner dans différents modes de fonctionnement et comprenant un ou plusieurs composants pouvant être basculés dans différents modes ayant des besoins en énergie différents en fonction du mode de fonctionnement de l'unité électronique, l'unité électronique comprenant au moins un processeur (23) et au moins une mémoire non volatile (24, 25) destinée à stocker un programme configurant l'au moins un processeur pour exécuter un procédé de l'une quelconque des revendications 1 à 10.

13. Unité électronique de la revendication 12, dans laquelle le ou les composants comprennent au moins un des éléments suivants :
- un dispositif de commande de capteur prévu pour une capture de dose ;
- un dispositif de commande principal prévu pour un enregistrement de dose et une commande de transmission de données ;
- au moins un composant d'interface de communication (27), en particulier un module de communication Bluetooth^{®}.

14. Dispositif d'administration de médicament (12) ou dispositif complémentaire d'administration de médicament comprenant l'unité électronique (10) de l'une quelconque des revendications 12 et 13.
